# EUROPEAN PATENT SPECIFICATION

(11) **EP 2 140 816 B1**
(45) Date of publication and mention of the grant of the patent: **10.02.2016**
(21) Application number: 09251704.4
(22) Date of filing: 01.07.2009
(51) Int. Cl.: A61B 17/02, A61B 17/70, A61F 2/46, A61M 25/09, A61M 29/00, A61B 17/32

(54) **Access and tissue modification systems**
Zugangs- und Gewebeänderungssystem
Systèmes d'accès et de modification des tissus

(30) Priority: 01.07.2008 US 77441 P
(43) Date of publication of application: 06.01.2010
(73) Proprietor: Baxano, Inc., Mountain View, CA 94043 (US)
(72) Inventor: Chung, Winnie, Mountain View California 94043 (US); Borgstrom, Amie, Mountain View California 94043 (US); Bleich, Jeffrey, Mountain View California 94043 (US); Arcenio, Greg, Mountain View California 94043 (US); Leguidleguid, Ronald, Mountain View California 94043 (US); Leguidleguid, Roy, Mountain View California 94043 (US); Schmitz, Gregory P., Mountain View California 94043 (US); Wallace, Michael P., Mountain View California 94043 (US)
(74) Representative: Potter Clarkson LLP

(56) References cited:
- WO-A-00/67651
- WO-A-99/21500
- WO-A-2005/009300
- WO-A-2007/008709
- WO-A-2008/157513
- US-A- 6 102 930
- US-A1- 2006 122 620
- US-A1- 2006 258 951
- US-A1- 2008 147 084

## Description

### BACKGROUND OF THE INVENTION

Minimally invasive surgical techniques typically include accessing the tissue through a small opening or port into the body. Minimally invasive procedures may include laparoscopic devices and remote-control manipulation of instruments with indirect observation of the surgical field through an endoscope or similar device, and may be carried out through the skin or through a body cavity or anatomical opening. This may result in shorter hospital stays, or allow outpatient treatment.

Unfortunately, the use of minimally-invasive techniques has often required a loss in control of the treatment device or implant, as the treatment sites are often deep within the body, proving both difficult to access, as well as difficult to manipulate the device when the body region is minimally invasively accessed. In particular, finding leverage to position or manipulate minimally invasive devices once deployed has proven extremely difficult. For example, most procedures are performed from a single (minimally invasive) opening through the body to access the treatment site. Thus, any devices or implants delivered through this opening must be controlled externally through the single opening. As a result, complex and expensive tools have been created to allow manipulation of distally-positioned devices or implants within the body.

Even in variations of minimally invasive procedures in which a second access port is used, coordination of the two access ports at the target has proven difficult, particularly when one or more devices are inserted through different access ports and required to meet at an internal site. Such minimally invasive techniques often require the additional use of visualization devices to guide and/or confirm device position and operation.

Finally, manipulation of implants and devices using any of these minimally invasive techniques has also proven difficult. For example, when treating small or enclosed body regions such as joints, or regions surrounded by sensitive non-target tissue, manipulation of a device or implant within this space has been limited by the ability to control the distal end of the device from a proximal position. When a single access point is used, the device or implant must generally be 'pushed' into position within or along an access device. An elongate member (e.g., a cannula or guide) may be used, and the control of an implant or other device depends on the configuration of the access elongate member. Thus, the application of force by the implant or treatment device may depend on the application of force from the proximal end, at some distance from the distal end where the implant or treatment device is located. This may lead to undesirable and dangerous kinking, bending, and torqueing of the access device and/or implant.

US 2006/0122620, which is considered to be the closest prior art, relates to systems and methods for stabilizing or adjusting the position of at least one spinal motion segment. The systems are implantable posterior to the spine and comprise expandable lateral members and transverse members for providing inner spinious distraction.

WO 00/67651 discloses a method and apparatus which utilizes an arcuate implant member for stabilizing adjacent vertebrae of the spine.

US 2006/0258951 relates to a tissue modification device and methods for locating neural tissue in a patient body.

WO 2005/009300 discloses a prosthesis to be inserted between the spinious processes of the vertebra through an incision tube. The prosthesis is capable of restricting movement of the vertebra when a torso extends backwards at a waist.

WO 99/21500 discloses a spine distraction implant (1000) which alleviates pain associated with spinal stenosis by expanding the volume in the spine canal and/or neural foramen. The implant provides a spinal extension stop (1016) while allowing freedom of spinal flexion.

US 2008/0147084 relates to a device for modifying tissue in a patient which includes an elongate body, a proximal handle, one or more tissue modifying members, a guidewire coupled with and extending from the body and a distal handle removeably coupleable with the guidewire outside the patient.

WO 2007/008709 discloses an apparatus which includes a projection having a tapered end portion configured to be inserted percutaneously through tissue. A measurement member is coupled to the projection and is configured to indicate a distance between adjacent anatomical structures.

US 6,102,930 discloses a device for accurately determining the lateral recess and foraminal volume prior to and following spinal decompression procedures to assess adequacy of decompression.

Described herein are methods, devices and systems for treating tissue by first placing a guidewire (or "pullwire") in position within the body, and then using the guidewire to position, anchor and/or treat the tissue. In general, these methods and systems are "bimanual" procedures, in which the implant or tissue modification device is controlled within the body from two separate locations outside of the body. The devices, methods and systems described herein may allow precise control and anchoring of one or more devices, and therefore precise treatment of tissue, and may address many of the issues raised above. Although the methods described herein may be particularly suitable for minimally invasive (e.g., percutaneous) treatment of tissue, they may also be used for open or semi-open treatments.

### SUMMARY OF THE INVENTION

According to the present invention, there is provided a system for inner spinous distraction, the system comprising:
an inner spinous distractor configured to be pulled into position through the inner spinous ligament between two spinous processes and to distract the two spinous processes;
a pullwire having a tissue-penetrating distal end and a coupler at the proximal end, the coupler configured to releasably couple to the inner spinous distractor so that the pullwire may be used to pull the inner spinous distractor into position; and
a cannulated probe configured to position the pullwire between two spinous processes.

Preferably, the system further comprises a sizer configured to couple to the proximal end of the pullwire so that it can be pulled between two spinous processes.

Conveniently, the system further comprises a distal handle configured to attach to the distal end of the pullwire and to secure the tissue-penetrating distal end of the pullwire.

Advantageously,, the system further comprises an inner spinous distractor delivery tool configured to hold the inner spinous distractor for delivery between two spinous processes, wherein the distal end of the delivery tool comprises a coupler for coupling to the proximal end of the pullwire and the proximal end of the inner spinous distractor delivery tool comprises a proximal handle.

Preferably, the system further comprises a lock for securing the inner spinous distractor in position between two spinous processes.

Also described herein, there is provided an electrical lead for pain management configured to be pulled into position distally and anchored distally and proximally, the device comprising:
an elongate body having a distal coupling region configured to couple to the proximal end of a pullwire;
a first anchoring element at the distal end configured to anchor the lead within the body;
a second anchoring element at the proximal end configured to anchor the lead within the body; and
a plurality of electrical contacts located between the proximal and distal anchors.

Preferably, the device further comprises a proximally-extending electrical connector configured to connect to an implantable pulse generator for applying energy to the plurality of electrical contacts.

Also described herein, there is provided a system for positioning and anchoring an electrical lead relative to a patient's spinal nerves, the system comprising:
an electrical lead comprising a distal connector configured to be used to distally pull the lead adjacent to a target spinal nerve tissue;
a pullwire having a tissue-penetrating distal end and a coupler at the proximal end, the coupler configured to couple to the distal connector of the electrical lead so that the pullwire may be used to pull the electrical lead into position; and
a cannulated probe having a curved distal end, the probe configured to position the pullwire adjacent to the target spinal nerve tissue.

Preferably, the system further comprises a neural localization device having a distal connector configured to couple to the coupler at the proximal end of the pullwire.

Conveniently, the system further comprises a distal handle configured to attach to the distal end of the pullwire and to secure the tissue-penetrating distal end of the pullwire.

In general, described herein are exemplary methods for precisely placing and/or manipulating devices within the body by first positioning a guidewire through the body from a first location, around a curved pathway, and out of the body through a second location, so that the distal and proximal ends of the guidewire extend from the body, then pulling a device into position using the guidewire. The device to be positioned within the body is coupled to the proximal end of the guidewire, and the device is pulled into the body by pulling on the distal end of the guidewire that extends from the body. The device may be bimanually manipulated by pulling the guidewire distantly, and an attachment to the device that extends proximally, allowing control of both the proximal and the distal ends. In this manner devices (and particularly implants such as innerspinous distracters, stimulating leads, and disc slings) may be positioned and/or manipulated within the body. Devices to modify tissue may also be positioned or manipulated so that a target tissue within the body is modified.

Devices and systems configured to be coupled to the proximal end of a pull guidewire (or "pullwire") are also described. In general, a system for pulling an implant or tissue modification device into position as described herein may include a probe for positioning a guidewire into position, a guidewire/pullwire, a handle for the guidewire/pullwire, and a device having a distal end configured to couple to the pullwire and be pulled into position by the pullwire. The devices or implants may be adapted for use with the pullwire. For example the distal end of the devices/implants may be configured to releaseably secure to the proximal end of the pullwire. Furthermore, the devices may be adapted so that the connection with the guidewire is sufficient to withstand a substantial amount of pulling force that may be applied when positioning or manipulating the device(s).

For example, the general devices and methods described herein may be used to position and/or manipulate devices involved in the treatment of any of the following conditions: positioning/implanting stimulator leads (including anchoring them) within the body, and especially within the lateral recess or foramen; treatment of chronic total occlusions, including retrograde treatment (e.g., pull through); placement of pedicle screw(s); accessing a facet joint for fusion (e.g., posterior lateral gutters), implantation, etc.; spinal fusions, including percutaneously pulling in a rod between the screws; discectomy; remove or repair of disc herniation; pain management, including delivery of drug depot (e.g., ribbon, pod, electrodes, etc.), and particularly placement within spinal regions such as the facet joint; treatment of spine tumors (e.g., cage); insertion/implantation of stem cells; implantation of interlaminar wires; rapid laminectomy (e.g., in/out technique); treatment of distal clavicle, including shoulder impingement; treatment of entrapment Syndrome (e.g., carpel tunnel); removal of tumors, osteophites, around rib cage, ribs; thoracotomy; treatment of bone spurs; treatment of knees, including positioning/implanting drugs depots (e.g., steroids) and resurfacing of the joint; resurfacing of joints generally (spinal, etc.), including resurfacing of cartilage and preparation of joint for implant(s); removal of adipose (fat) tissue (e.g., liposuction); reconstructive surgeries (e.g., rhinoplasty, etc.); and the like.

Described below are particular examples, including devices adapted for use with these examples that illustrate methods of performing such treatments and therapies. For example, described herein are methods of performing inner spinous distraction. Inner spinous distraction may be performed as part of another procedure, including a spinal decompression procedure, since it may enhance access to regions of the spine requiring decompression.

Also described herein are exemplary devices and methods for implanting and anchoring an electrical lead. An electrical lead may be used to help treat chronic pain. The devices and methods described herein may allow precise implantation and anchoring of a lead. Adequate anchoring of implants (such as leads) is critical to prevent migration and eventual failure of these devices.

Also described are exemplary methods of treating spinal bone such as facet joints. For example, described herein are methods of resurfacing adjacent facet joints as part of a fusion procedure.

In another variation, method of performing discectomy are also described, which may also be performed as part of a separate procedure, or as part of a decompression procedure.

For example, described herein are methods for placing an inner spinous distractor within a body using a pullwire having a tissue-penetrating distal end and a proximal end. These exemplary methods may include: extending a pullwire across an inner spinous ligament between two spinous processes so that the proximal end of the pullwire extends from a first position outside of the body, and the distal end of the pull wire extends from a second position outside of the body; and pulling the distal end of the pullwire to pull a spinous process distractor from the first position into the inner spinous ligament between the two spinous processes.

The method may also include the step of coupling the proximal end of the pullwire to a distal end of the spinous process distractor. For example, the method may include coupling the proximal end of the pullwire to a distal end of a spinous process distractor delivery device. The step of extending the pullwire may include percutaneously passing the pullwire through the body from a first opening in the body at the first position to a second opening in body at the second position.

The method may also include detaching the distal end of the pullwire from the spinous process distractor. The pullwire may then be removed from body; in some variations the pullwire may remain coupled to a portion of the spinous process detractor delivery device, which may be removed with the pullwire.

The method may also include pulling a sizer between the two spinous processes using the pullwire. The sizer may be used to determine the appropriate size spinous process distractor to use.

In some variations the method also includes locking the spinous process distractor in position between the two spinous processes. The method may also include expanding the spinous processes distractor.

The step of extending a pullwire may include inserting a curved, cannulated probe between the spinous processes and passing the pullwire through the cannulated probe to extend from the distal end and out of the second opening out of the body. In some variations, the probe may include an outer cannula and an inner cannula that is configure to be extend from the distal end of the outer cannula in a curved pathway.

Also described herein are methods of placing an inter spinous distractor within a body using a pullwire having a tissue-penetrating distal end and a proximal end, the method comprising inserting a curved, cannulated probe between two spinous processes so that the tip of the probe extends in a curved pathway through the inner spinous ligament; extending a pullwire through the probe so that a distal end of the pullwire extends out of the body while the proximal end extends from the body proximally; removing the probe while leaving the pullwire in position across the spinous ligament; and pulling the distal end of the pullwire to pull a spinous process distractor between the two spinous processes.

Also described herein are systems for inner spinous distraction, the system comprising: an inner spinous distractor configured to be pulled into position trough the inner spinous ligament between two spinous processes and to distract the two spinous processes; a pullwire having a tissue-penetrating distal end and a coupler at the proximal end, the coupler configured to couple to the inner spinous distractor so that the pullwire may be used to pull the inner spinous distractor into position; and a cannulated probe, having a curved distal end, the probe configured to position the pullwire between two spinous processes.

In some variations, the system also includes a sizer configured to couple to the proximal end of the pullwire so that it can be pulled between two spinous processes.

The system may also include a distal handle configured to attach to the distal end of the pullwire and to secure the tissue-penetrating distal end of the pullwire.

In some variations the system also includes an inner spinous distractor delivery tool configured to hold the inner spinous detractor for delivery between two spinous processes, wherein the distal end of the delivery tool comprises a coupler for coupling to the proximal end of the pullwire and the proximal end of the inner spinous distractor delivery tool comprises a proximal handle.

The system may also include a lock for securing the inner spinous distractor in position between two spinous processes.

Also described herein are exemplary methods of implanting a lead for electrical stimulation adjunct to a target nerve tissue, the method comprising: extending a pullwire adjacent to the target nerve tissue so that the proximal end of the pullwire extends from a first position outside of the body, and the distal end of the pull wire extends from a second position outside of the body; coupling the distal end of the lead to the proximal end of the pullwire; and pulling the distal end of the pullwire to pull an electrical lead from the first position so that the lead is adjacent to the target nerve tissue.

The method may also include the step of anchoring the proximal and distal end of the lead. For example, the step of anchoring the proximal and distal end of the lead may comprise expanding an expandable member, or inflating a balloon.

The method may also include de-coupling the distal end of the lead from the proximal end of the pullwire and withdrawing the pullwire distally from the body.

The step of extending the pullwire may include passing the pullwire over a spinal pedicle. In some variations, the step of extending the pullwire comprises passing the pullwire down the lateral recess between two spinal lamina.

The method may also include confirming the position of the target nerve relative to the path of the guidewire. For example, a nerve localization device (including a plurality of electrodes for stimulating nerves that are immediately near the localization device) may be used, for example, by pulling the neural localisation device through the tissue using the pullwire.

Also described herein are exemplary electrical leads for pain management that are configured to be pulled into position distally and anchored distally and proximally. For example, such a lead may include: an elongate body having a distal coupling region configured to couple to the proximal end of a pullwire; a first anchoring element at the distal end configured to anchor the lead within the body, a second anchoring element at the proximal end configured to anchor the lead within the body; and a plurality of electrical contacts located between the proximal and distal anchors.

The electrical lead devices may also include a proximally-extending electrical connector configured to connect to an implantable pulse generator for applying energy to the plurality of electrical contacts.

Also described herein are systems for positioning and anchoring an electrical lead relative to a patient's spinal nerves, the system comprising: an electrical lead comprising a distal connector configured to be used to distally pull the lead adjacent to a target spinal nerve tissue; a pullwire having a tissue-penetrating distal end and a coupler at the proximal end, the coupler configured to couple to the distal connector of the electrical lead so that the pullwire may be used to pull the electrical lead into position; and a cannulated probe having a curved distal end, the probe configured to position the pullwire adjacent to the target spinal nerve tissue.

In some variations the system includes a neural localization device having a distal connector configured to couple to the coupler at the proximal end of the pullwire. In some variations the system further comprises a distal handle configured to attach to the distal end of the pullwire and to secure the tissue-penetrating distal end of the pullwire.

Also described herein are methods of fusing a facet joint using a bimanual treatment device. For example, a method of fusing a facet joint using a bimanual treatment device the method may include the steps of: extending a pullwire between two spinous processes so that the proximal end of the pullwire extends from a first position outside of the body, and the distal end of the pull wire extends from a second position outside of the body; coupling the distal end of a facet joint modifying treatment device to the proximal end of the pullwire; pulling the distal end of the pullwire to pull the facet joint modifying treatment device from the first position so that the facet joint modifying treatment device is adjacent to the facet joint; and reciprocating the facet joint modifying treatment device by pulling distally on the pullwire and proximally on the facet joint modifying treatment device.

The method may also include the step of applying a filling material between the facet joint Filling materials may include cement (e.g., bone cement), graft materials, or the line. The method may also include the step of inserting a support between the facet joint by pulling the cage in distally using the pullwire. For example, the support may comprise a cage, and/or an expandable member.

In some variations the method includes the step of cutting the superior spinous process of the facet.

Any appropriate facet joint modifying treatment device may be used, including a facet joint modifying treatment device having a bone-cutting surface.

### BRIEF DESCRIPTION OF THE DRAWINGS

FIG. 1 shows a median sagittal section of two lumbar vertebra and their ligaments.
FIG. 2 is a transverse section through the spine, showing the lamina and the superior and transverse processes.
FIGS. 3A-3F illustrate one variation ofa system with tools for bimanual treatment of tissue; this variation includes: two variations of a guidewire or pullwire positioning probe tool (3A and 3B), a flexible neural localization tool (3C), a tissue modification tool (3D), a removable guidewire handle (3E), and a guidewire (3F).
FIGS. 4A-4J show the components of one embodiment of an inner spinous distraction access and decompression kit.
FIGS. 5A-5J illustrate an embodiment of inserting an IPD to distract a patient's spine; FIGS. 5K-5R illustrate a method of decompressing a region of the spine that has been distracted after insertion of the IPD.
FIG. 6 shows one example of a spinal cord stimulator system implanted into a patient. This spinal cord stimulator system includes a lead and may be used to treat pain.
FIG. 7 shows a schematic of one potential pathway for implanting an electrical lead using the pullwire systems described herein; be this example the pullwire is inserted so that il extends above a pedicle.
FIG. 8 shows another pathway that may be used to position and/or anchor a stimulator lead.
FIG. 9 illustrates one variation of a lead that is adapted for pulling into position using a pullwire.
FIG. 10A illustrates a facet joint 1005 including the superior and inferior surfaces
FIG. 10B shows another portion of a spine including a facet joint 1011 that may be fused as described herein.
FIGS. 11A-11C illustrate variations of joint treatment devices. In FIG. 11A, the treatment device includes a front and a back articulating surface that can be drawn across the joint surfaces to roughen them; FIGS. 11B-11D show different cross-sections through joint treatment devices, and FIG. 11E illustrates another variation of a joint treatment device. Any of these joint treatment devices may be facet joint treatment devices.
FIG. 12A illustrates a cross-section through one variation of a facet-joint modifying device that includes two bone-sawing elements.
FIG. 12B illustrates a cross-section through one portion of the device having a breakable spacer.
FIG. 12C shows a top view of one variation of a facet-joint modifying device configure to perform a facetectomy.
FIG. 13A shows one variation of a tissue treatment device having a semi-rigid or stiff and curved shape with a tissue cutting (e.g., serrated) edge on one or more sides. The device may be delivered in an uncurled (flexible) configuration, but may be curved into a more rigid form. Similarly FIG. 13B shows another variations of a semi-rigid curved tissue treatment device.
FIG. 14A shows a cross-section though a portion of the spine, indicating the more dense cortical bone regions.
FIGS. 14B and 14C illustrate one variation of a PLIF-type procedure that is made more effective using the pullwire techniques described herein.

### DETAILED DESCRIPTION OF THE INVENTION

As mentioned, described herein are exemplary devices, systems and method for treating tissue by first placing a guidewire (or "pullwire") in position within the body, and then using the guidewire to position, anchor and/or treat the tissue. In general, these methods and systems are "bimanual" procedures, in which the implant or tissue modification device is controlled within the body from two separate locations outside of the body, and by manipulating the implant/device from both the distal and proximal ends.

These systems and methods may be particularly useful for percutaneous treatments of one or more body region. However, it should be understood than any of the devices, methods and systems described herein may be used as part of an "open" surgical procedure in which access to a body region is created through an opening in the tissue (e.g., by removal of tissue). Any of the systems and devices described may be performed as part of a procedure that is at least partially open. Partially percutaneous procedures may also be performed using these devices, systems and methods.

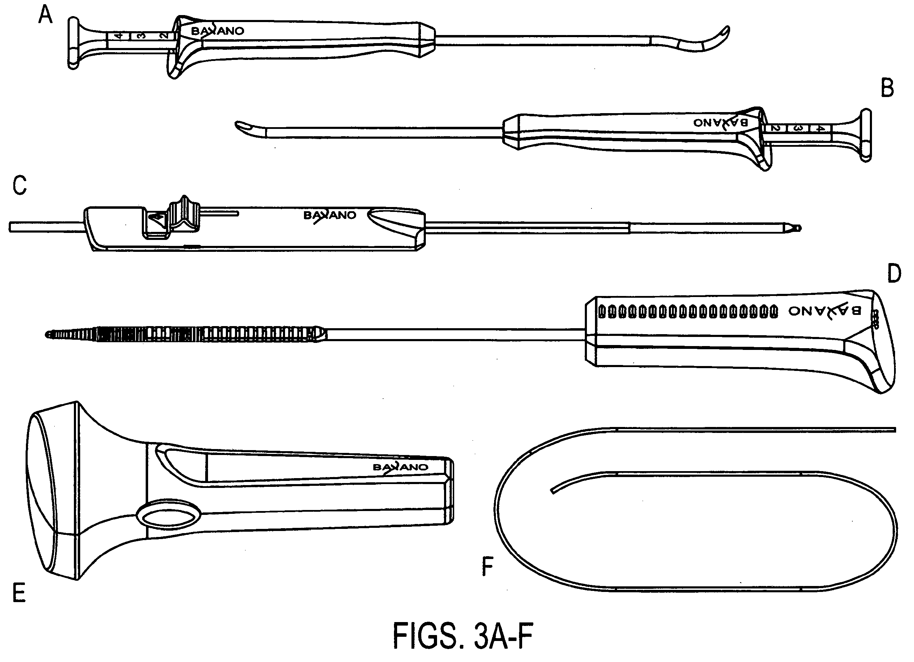

FIGS. 3A-3F illustrate components a system that may be used to treat tissue as described herein. The components illustrated in FIGS. 3A-3F include: two variations of probes (3A and 3B) that may be used to position a guidewire (or pullwire) in the tissue, a neural localization device (FIG. 3C) that is configured to be coupled to the proximal end of a guidewire, a tissue modification device (FIG. 3D) that is configured to scrape or cut tissue and be coupled distally to the proximal end of a guidewire, as well as a guidewire (FIG. 3F) and a handle that may be secured to the distal end of the guidewire (FIG. 3E) allowing manipulation of the distal end of the guidewire/pullwire.

In particular, the guidewire, guidewire handle and placement probes (FIGS. 3A, 3B, 3E and 3F) may be used with one or more additional components to treat a patient, as illustrated in the examples below. In general, these devices may be used to place the guidewire in position within the body so that the (often sharp) distal end of the guidewire extends from the body, and the distal end of the guidewire (which may be adapted to couple to another device so that force can be applied by pulling on the guidewire) extends from a second location in the body.

As mentioned, the proximal end of the guidewire may be adapted to couple to another device or devices. Examples of guidewires that may be used are described, for example, in US 2008-0086114 A1, titled "TISSUE ACCESS GUIDEWIRE SYSTEM AND METHOD" (filed 8/29/2006), and US 2008-0275458 A1, titled "GUIDEWIRE EXCHANGE SYSTEMS TO TREAT SPINAL STENOSIS" (filed) 5/27/2008. The distal end of the implant or device to be positioned and/or manipulated may also be adapted to couple to the guidewire as described.

Described herein is a guidewire- or pullwire-based system for distracting a bone or region including bone. These methods may be used to distract bone to treat a compression fracture (e.g., a spinal compression fracture) or to separate bones or bony regions to allow access for further treatment. For example, an access system such as a pullwire-based system can be used to deliver a percutaneous distraction system for distracting the inner spinous process and delivering an inner spinous process distraction device (IPD). Thus, in some variations, described herein are percutaneous inner spinous distraction access and decompression systems, devices and methods of using them.

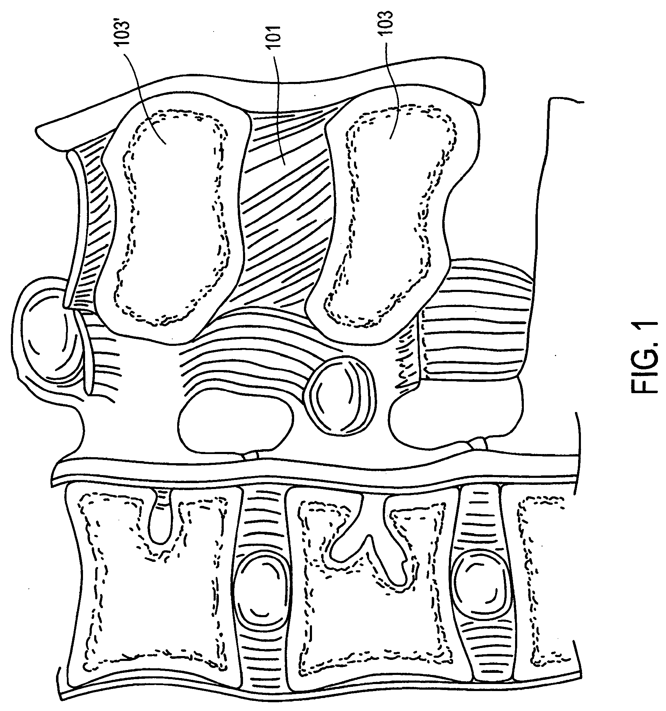

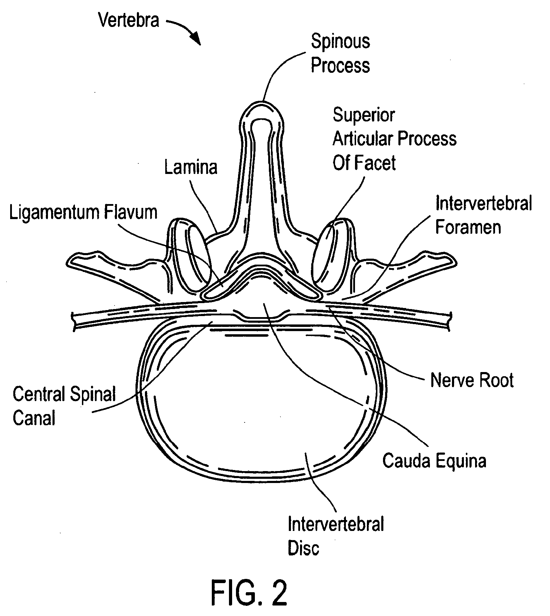

FIGS. 1 and 2 illustrate sections through a normal spine region, including the inter spinous process region. FIG. 1 shows a median sagital section of two lumbar vertebra and their ligaments. In FIG. 1, the section through a region of spine illustrates the inter-spinal ligament 101 connected between two spinous processes 103, 103'. FIG. 2 illustrates a transverse section through the spine, showing the lamina and the superior and transverse processes.

As described in greater detail below, an inner spinous process distraction device (IPD) may be inserted using the pullwire system. This method of distracting the spinous processes may be used in conjunction with (or as part of) a procedure for decompressing the spine including delivering a transforaminal guide through the foramen. With the IPD holding a foramina open, a decompression procedure can be performed.

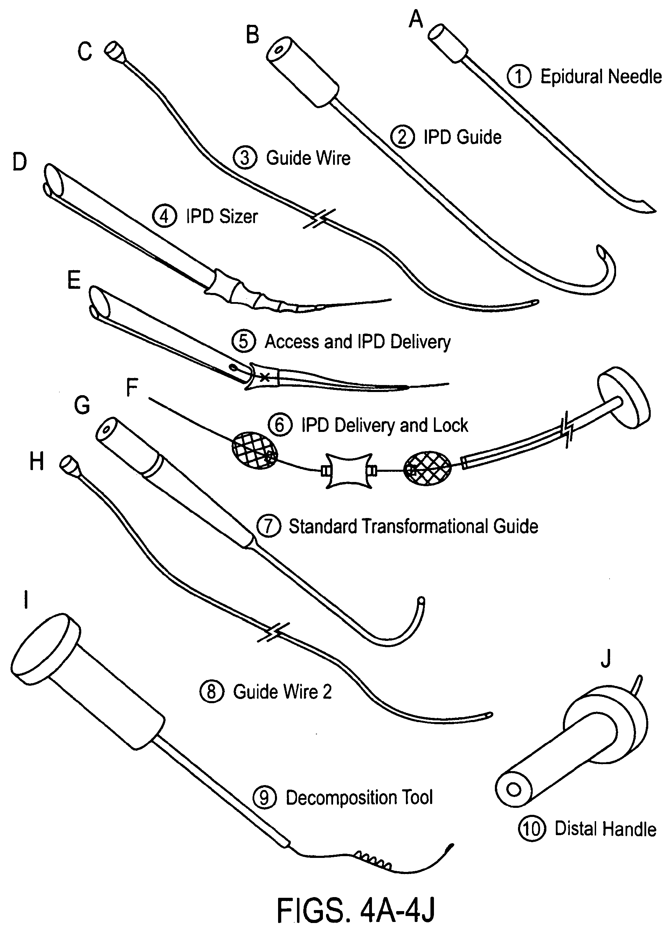

One embodiment of an inner spinous distraction access and decompression kit is shown in FIG. 4A-4J. Some of the components illustrated in FIGS. 4A-4J are redundant, and may be omitted. Many of these elements are also similar or identical to the elements shown in FIG. 3A-3F, and may be used with these elements. For example, a system for inserting a IPD may include a probe for inserting and positioning a guidewire/pullwire, a pull wire that is adapted to couple to the distal end of a IPD (or a carrier for the IPD), an IPD, and an IPD delivery tool that holds the IPD and may include a proximal handle or manipulator. Examples of these elements are shown in FIG. 4A and 4B (probes for positioning the guidewire, including an epidural needle and a curved IPD guide), FIG. 4C (pullwire/guidewire), and FIG. 4E (IPD delivery tool or carrier with attached IPD). Additional components of this system may also include a sizer (FIG. 4D), a lock or locker for securing the IPD in position (FIG. 4F), and a handle for the distal end of the pullwire/guidewire (FIG. 4J). In addition, the system shown in FIGS. 4A-4J may also include elements that may be used for the decompression of other spinal regions, including the foramen of the spine. For example, the system may include an additional probe (FIG. 4G) that is shaped and sized for transforamenal access, as well as a tissue modification member (FIG. 4I), and an additional guidewire/pullwire (FIG. 4H).

In general, the probe element is an elongate, somewhat rigid and cannulated structure. In some variations the guide includes a curved or curvable distal end region. For example, the probe may include an inner cannula that can be extend distally from the outer cannula; the inner cannula may curve as it is extend, allowing steering of the device around a body region. In general, the pullwire/guidewire may be extended through the probe, into the body, around a target tissue region, and then allowed to pass back through and out of the body from a second region. More than one probe may be used in any of the methods described herein. For example, probes having different curvatures or lengths may be used in any of these methods.

For example, in FIGS. 4A-4J, the elements shown in FIGS. 4A-4F may be used to deliver an IPD into the body. Elements shown in FIGS. 4G-J may be used as adjuncts to the IPD system for accessing and decompressing the foramen.

As mentioned, any appropriate guidewire may be used, particularly those including a tissue-penetrating distal end and a proximal end that is configured to releasably couple to the distal end of an implant or device (e.g., the IPD delivery device shown in FIG. 4F). FIGS. 4C and 4H both illustrate pullwires or guidewires that are so adapted. For example, the proximal end may include a lip or rim (e.g., a ball, cylinder, etc.) that may be coupled with a receiver on the device or implant.

A sizer may be used to determine what size implant (e.g., IPD) is appropriate for use within the patient. Examples of sizers that may be used are illustrated in US 2008-0312660 A1 (filed 6/16/08). One variation is shown in FIG. 4D, and includes a distal end that couples to the guidewire so that it can be pulled distally into the inner spinous space (e.g., between the inferior and superior processes). Based on how far it can be pulled into the space, the size of the opening, and therefore an appropriately sized implant, may be determined.

In the IPD system shown in FIGS. 4A-4J, the IPD is attached to a delivery device shown in FIG. 4E. In this example, the IPD sits in a portion of the delivery device so that the delivery device may be coupled distally to the guidewire after it has been positioned and pulled in to place. The proximal end of the IPD delivery device is configured to extend out of the device, and may include a release control for releasing the IPD once it has been placed within the body (e.g., between the spinal processes as described below). For example, the IPD delivery device may include a wire or cable connecting the proximal end (elongate proximal region) and the distal end through the implant (IPD). Triggering release of the IPD once it has been positioned will release the distal end coupled to the guide wire/pull wire from the proximal end coupled to a proximal handle. Releasing the distal end may allow it to be withdrawn from the body by pulling on the guidewire/pullwire (distally) and the rest of the IPD delivery device may be withdrawn proximally. The IPD implant is left behind in position.

The implant may then be secured in place. For example, FIG. 4F illustrates an IPD locking device. The locking device includes one or more expandable regions that either connects to the implant (IPD) to hold it into place, or they alter the shape of the implant to hold it in place. In some variations, the device is self-locking. For example, the device may include a shape or structure that expands after being implanted, preventing it from dislodging or migrating. In some variations one or more "locks" or anchors may be attached or extended from the device to hold it in place.

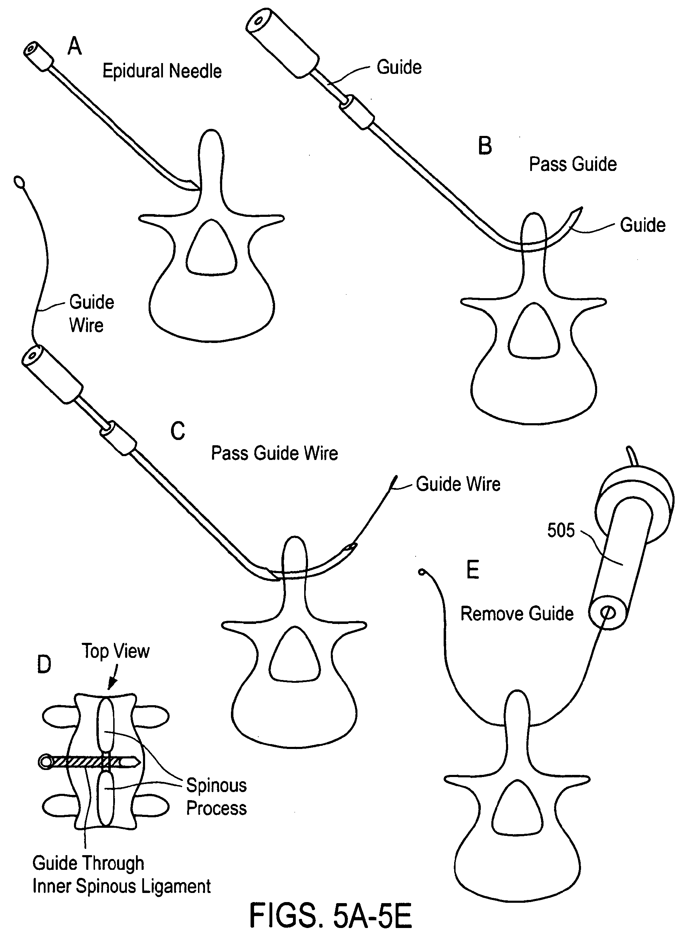

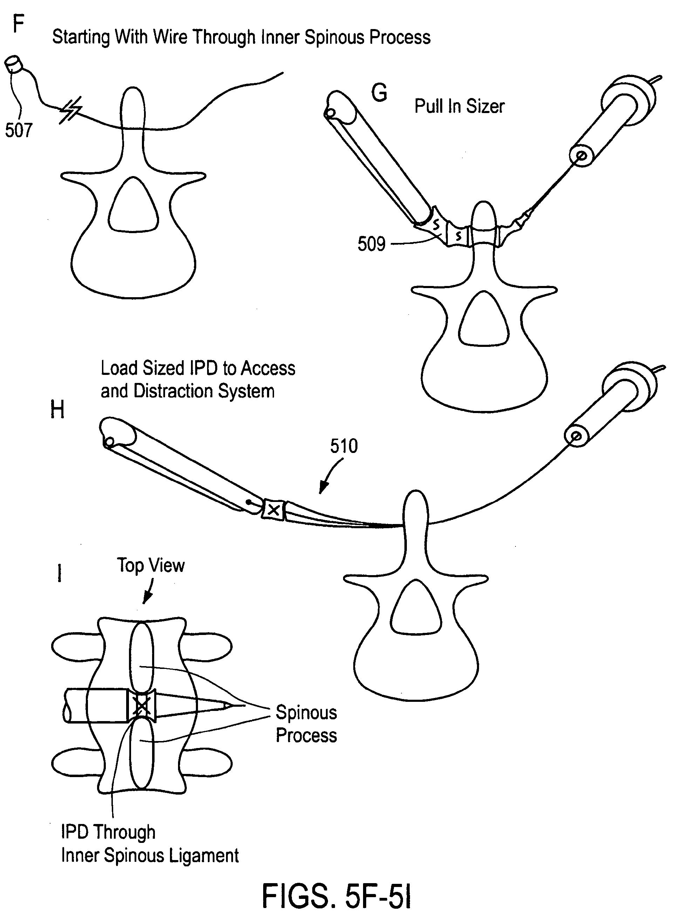

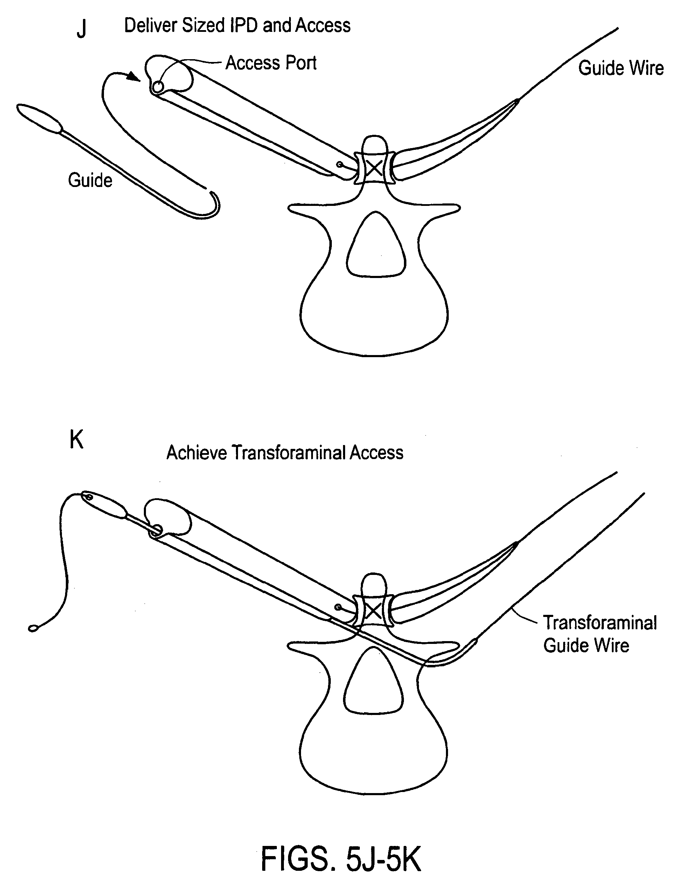

FIGS. 5A-5J illustrates one variation of inserting an IPD to distract a patient's spine; FIGS. 5K-5R illustrate an exemplary method of decompressing a region of the spine that has been distracted after insertion of the IPD. In this example, the IPD is delivered through the inner spinous process ligament For example, in FIG. 5A, the guide (shown here as an epidural needle) is inserted from outside of the patient to the inner spinous process ligament. In FIGS. 5A-5J, the spinal structures illustrated resemble though shown in slightly more detail in FIGS. 1 and 2. In FIG. 5B, a second probe (or portion of the probe) is then extended coaxially from the first probe is extended through the ligament In this example, the inner probe cannula has a tissue-penetrating tip that passes through and curves back dorsally, as shown. A sharpened guidewire may then be passed through the probe, as shown in FIG. 5C. FIG. 5D shows a top view of a portion of the probe passing between the spinous processes and through the inner spinous ligament The probe (inner and outer members) may then be removed, leaving the guidewire/pullwire extended through the ligament, as shown in FIGS. 5E and 5F. In this example, the guidewire extends both distally and proximally from a patient's body. The proximal end of the guidewire includes a coupling member 507 for coupling to the distal end of a device, as described above. A distal handle 505 may than be attached to the distal end of the guidewire, as shown. In some variations, this distal handle 505 includes a capture mechanism for capturing the sharp distal end of the pullwire.

With the guidewire through the inner spinous process ligament, a sizer 509 can then pulled through with the distal handle, shown in FIG. 5G. After sizing the distraction space, the appropriately sized IPD can be inserted between the inner spinous processes using the IPD delivery tool 510, as shown in FIG. 5H. The IPD delivery tool 510 includes an IPD and is distally coupled to the proximal end of the guidewire, as shown pulling on the distal handle 505 connected to the guidewire pulls the IPD in the delivery tool through the ligament until it is positioned as desired (e.g., shown in FIG. 5I in top view), between the spinous processes and within the ligament. The IPD may be positioned by bimanually manipulating the IPD delivery device. For example, the device may be pulled distally by pulling on the distal handle, or proximally by pulling on the proximal end (handle) of the IPD delivery device.

Once the IPD is in position, the IPD delivery device may be decoupled from the IPD, so that the distal region of the delivery device can be withdrawn distally (by pulling on the guidewire) and the proximal portion can be withdrawn proximally, leaving the device in place. In some variations, the IPD may be locked into position either before, after or during the removal of the IPD delivery device.

Alternatively, FIGS. 5J-5R illustrate a variation in which the IPD implantation is used as part of a decompression procedure. In this example, the first guidewire remains attached to the IPD delivery device even after the IPD has been positioned. For example, in FIG. 5I, the proximal handle of the IDP delivery device may be used as a guide for delivering a probe to a different region of the spine. Thus, in this configuration, the spinal decompression portion of the procedure may be performed through the same patient entry point as the IPD (although the probe may be delivered through a second entry point, as well). The probe may be passed through the body to the spine, into the epidural space, and out of the foramen, as illustrated in FIG. 5K, to achieve transforaminal access. Some means of determining the entry into the epidural space may be used (i.e. a syringe and loss of resistance technique). After the probe is positioned, the guidewire may be passed through the probe, as illustrated in FIG. 5K. Neural localization may be used during the passing of the guide to confirm the guide is above the nerve root before passing the guide wire. The probe used to position the second guidewire may be a different probe than the one used to position the first guidewire, or it may be the same probe.

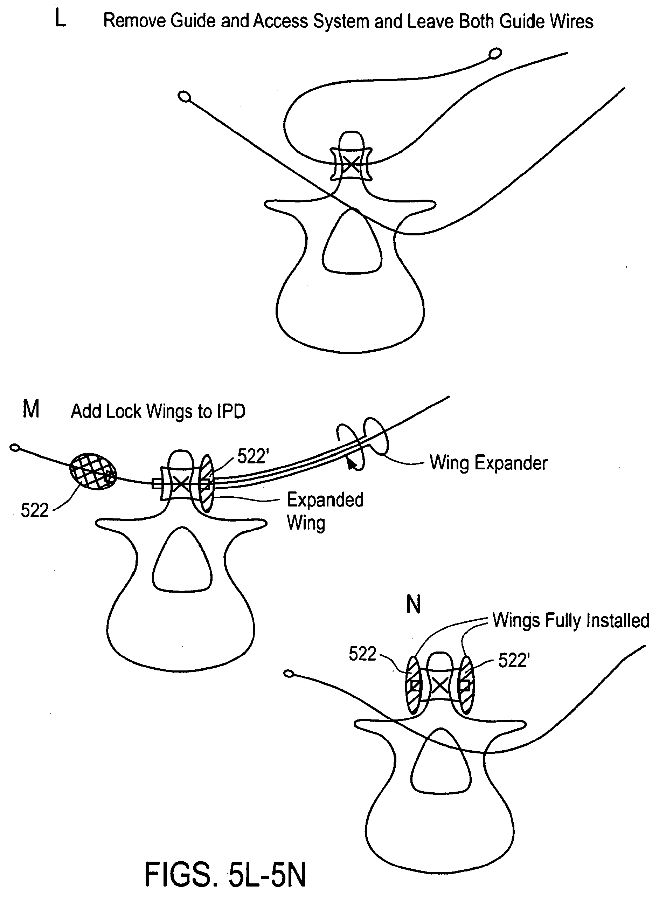

After accessing the foramen and passing a second guide wire, both the IPD delivery and foraminal access systems may be removed, as shown in FIG. 5L. In this variation, a guidewire may be left behind in both cases. In some variations a third guidewire may be passed through the IPD as the first guidewire (and the distal end of the IPD delivery device) is removed. For example, the IPD delivery device may include a passage or channel for a third guidewire delivery device; the distal end of the third guidewire may be releasably coupled to the inside of the distal end of the IPD delivery device. As it is withdrawn from the patient distally, the third guidewire is pulled through. Alternatively, in some variations the first guidewire remains in place as the IPD delivery device is removed proximally (e.g., pulling the proximal end of the guidewire proximally through the IPD and proximally out of the patient.

In the example, illustrates in FIG. 5M, the IPD is then looked in to position. For example, the IPD may be laterally secured by locking expandable wings 522, 522' in place on either side of the IPD. The wings can be made from a preset shape memory material that expands after placing or stainless steel plastic that is cold worked during installation, or non-expandable using solid metal or plastic. In some variations, the device may be locked in place by extending one or more anchoring structures (e.g., struts) from the IPD. The device may be anchored to or against the bone (spinous processes). FIG. 5N illustrates the IPD laterally anchored into position by the expandable wings 522, 522'. In some variations, the anchors may be deployed by deforming a portion of the IPD so that it secures the IPD (e.g., by expansion) in position. In some variations, the IPD may include an inflatable region that may be filled (e.g., from a proximal port) with a filler, including bone cement or other materials.

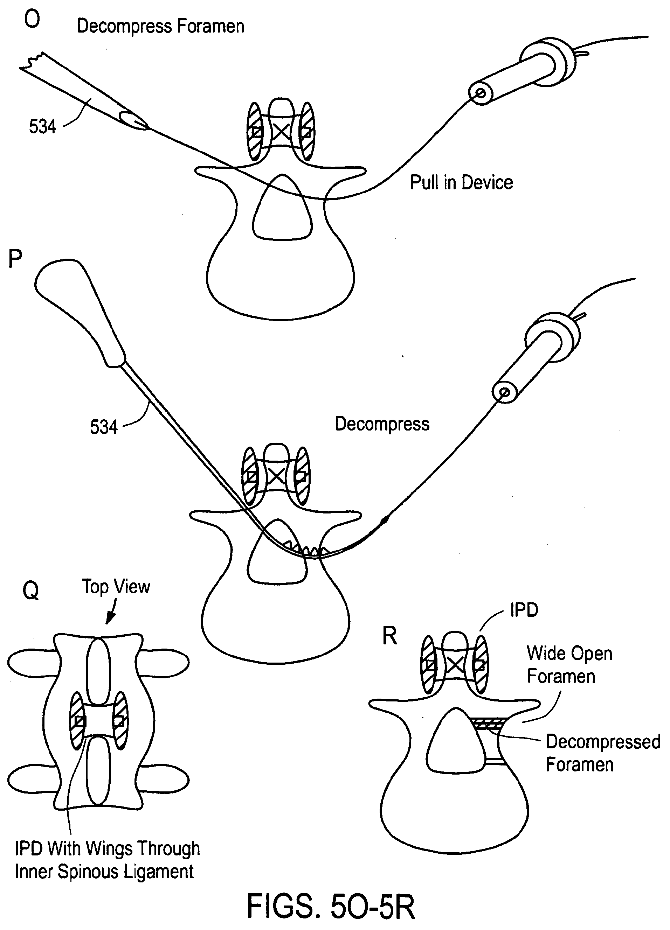

Thereafter, the foramen may be decompressed as illustrated in FIGS. 5O to 5R. For example, a tissue modification device may be pulled into the spinal foramen using the second guidewire, as illustrated in FIG. 5O. In this example, the distal handle is attached to the second guidewire and used to pull a tissue modification device 534. In this example, the tissue modification device includes a flexible distal end that includes a tissue modifying surface (e.g., a sharp or bladed surface) to remove tissue and thereby decompresses the foramen. The tissue modification device may include tissue capture for removing cut tissue. The tissue modification device may also include a proximal handle, allowing it to be manipulated proximally as well as distally, using the distal handle. In this example, the tissue modification device may be bimanually manipulated (moved back and forth proximally and distally) to decompress the tissue, as shown in FIG. 5P. Meanwhile, the IPD remains anchored, distracting the spinous processes, as illustrated in FIG. 5Q. This distraction may enhance access to the foramen, and thereby enhance the decompression. Once the decompression is complete, the tissue modification device 534 may be removed, and the decompression is completed as illustrated in FIG. 5R.

The method of distracting the processes and also of decompressing using this decompression may have many advantages over existing methods. As described above, the method (and variations of this method) allows percutaneous delivery for both IPD and decompression systems. As mentioned, however these methods and tools may also be used in an open (or partially open) procedure. In addition, the decompression and distraction may be achieved through same percutaneous entry point, or though different entry points.

One substantial advantage over existing methods of inserting the IPD and distracting the bone is that the distraction device is inserted by pulling (either pulling distally or pulling both distally and proximally). Existing method require pushing, which may be more difficult, particularly given curved or bent pathways through the body. In addition, pushing may require more force, and may also risk damaging surrounding tissue. Pulling to distract the bone achieves a mechanical advantage in part because a long flexible taper may be included at the distal and of the delivery device that is designed to allow it to make tight turns, allowing for straight posterior delivery.

In some variations, the distraction may be performed using an expandable or inflatable device that may be inserted either acutely or long-term. For example, an inflatable device may be pulled into position using the guidewire/pullwire as described above, and (once positioned) may be inflated or filled with a material, including a bone cement or other material (bone chips, etc.). Once inflated, the delivery device may be decoupled, leaving the inflated ("balloon" or fillable sleeve) in place. Alternatively, the device may be deflated/emptied and removed.

The exemplary methods described herein may also include visualization. For example, any of the steps described herein may include one or more visualization steps. Indicators, including radioopaque, ultrasound-visible, or other markers may be included on any of the devices described, including in particular the sizer and implant(s). The bimanual methods described herein also allow tactile feedback For example, tactile placement may be used to select the distraction size using the spacer (to feel how wide/narrow the opening to be distracted is).

In addition to methods of implanting distracters and other tissue-modifying devices, the methods and systems described herein may also be used to position and implant, including anchoring other devices, including electrical leads.

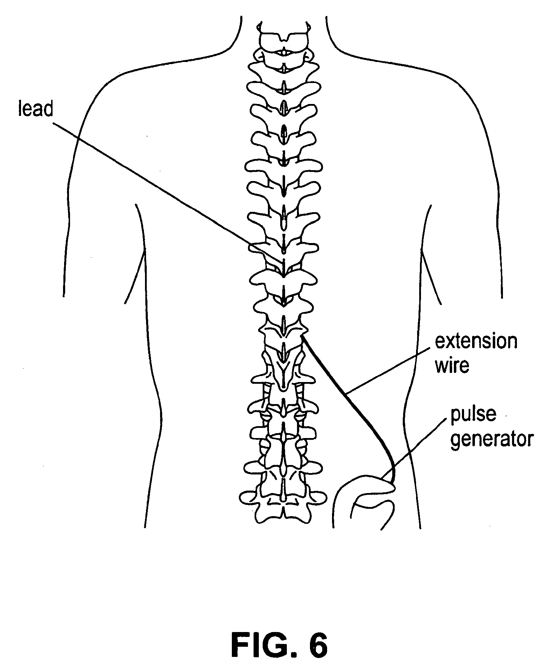

Electrical leads may be used to treat pain, particularly limb pain that is otherwise irresolvable. For example, a spinal cord stimulator, also known as a dorsal column stimulator, may include one or more leads that are implantable and used to treat chronic neurological pain. Once positioned within the body, the electrical lead may provide electric impulses to alter the perception of pain. The lead is typically implanted into the epidural space either by percutaneous approach or by surgical laminectomy or laminotomy. A pulse generator or RF receiver may then be implanted in the abdomen or buttocks, and a wire harness connects the lead to the pulse generator. For example, FIG. 6 illustrates one example of a spinal cord stimulator system that may be used to treat pain.

One problem with existing leads used for pain management is the necessary to ensure that the leads do not migrate substantially, and are placed in the correct portion of the body (spine) for optimal treatment The methods and systems described herein may be used to both position and anchor a lead, and may allow anchoring of both the proximal and distal end of the lead. For example, the methods described herein may allow anchoring of the lead to the spinous processes, to the lamina, within the lateral recess, within the foramen, or the like, so that the lead can be positioned appropriately near a neural target such as a spinal ganglion, nerve root, etc. As mentioned, this anchoring may allow reduced risk of migration of the lead.

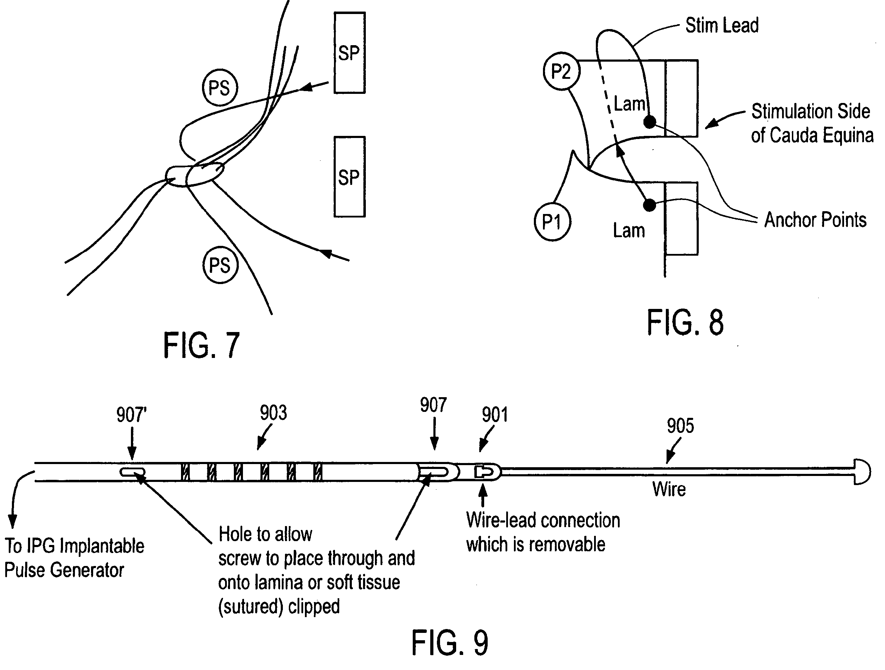

To place and anchor the stimulation lead, the system described above (e.g., in FIGS. 3A-3F) may be used to first position the guidewire adjacent to the implantation site. For example, one or more probes may be used to position create a pathway from a proximal insertion site adjacent to the target implantation site. A probe having an outer cannula may be inserted near the target implantation site (e.g., near a spinal target such as the nerve root or ganglion), similar to the method described above for spinal decompression. FIG. 7 shows a schematic of one potential pathway extending above a pedicle. The pathway allows the lead to be positioned near (adjacent to) a spinal ganglion, and past two or more spinous processes to which it can be anchored proximally and/or distally. Alternatively, the pathway may extend through the lateral recess and/or foramen so that the lead can be secured within the foramen.

For example, a cannulated probe may be inserted through the foramen, so that the distal end of the probe points towards an exit point out of the body; a guidewire or pullwire having a sharp or tissue-penetrating distal tip can then be inserted through the probe around the ganglion or other target nerve region, and out of the patient The probe allows the guidewire/pullwire to pass into the subject and around the target region. When the guidewire/pullwire exits the probe, it continues to extend from the probe in a substantially straight pathway until it extends from the subject, forming a second (e.g., a distal) exit point After the distal end of the guidewire/pullwire has exited the patient, it may be secured with a distal handle, as mentioned above, and the probe may be removed.

In some variations, the position of the guidewire/pullwire may be confirmed by using a neural localization device (as shown in FIG. 3C). In some variations, the neural localization device includes one or more electrodes that may be used to applying energy to stimulate nearby nerves; this stimulation can be detected when the neural localization device is sufficiently near the target neural tissue.

FIG. 8 shows another pathway that may be used to position and/or anchor a stimulator lead. In FIG. 8 the guidewire is positioned up or down the lateral recess from one lamina to another lamina. The lead may be anchored to the lamina so that the lead can apply simulation to the appropriate region of the canda equina. In this example the probe may form a channel for the guidewire/pullwire that extends between and around the lamina as indicated.

One the guidewire/pullwire has been positioned near the appropriate target, it may be used to pull the lead into position. FIG. 9 illustrates one variation of a lead that is adapted for pulling into position using a pullwire. Any appropriate lead may be used. For example, the lead may be a paddle lead, a round (or cylindrical) lead, or the like. In FIG. 9, the lead includes a distal coupling region 901 configured to releasably couple to the proximal end of the guidewire/pullwire as mentioned above. FIG. 9 shows the lead coupled to a guidewire 905 at the distale end. At either end of the stimulation electrode region 903 (shown as band electrodes), are distal and proximal anchor regions 907,907'. These anchor regions may include anchors such as sharp hooks or prongs that may be extended from the lead. For example, a lead anchor may be a superelastic or shape memory material that can be extended from the lead once it has been positioned. A control or release at the proximal end may control release of the anchor(s). In some variations the anchor region includes an expandable anchor, such as an inflatable or fillable member that may be expanded to secure the lead at either or both ends. In some variations an expandable member may be used to extend a hook or other anchor. In some variations the anchor regions include holes or openings through with an additional anchor (e.g., screw, hook, etc.) may be positioned. Alternatively, the lead may include integrated screws that may be used to attach the lead.

The proximal end of the lead shown in FIG. 9 includes a connector to an implantable pulse generator (IPG) that may be positioned elsewhere in the body. The lead maybe secured proximally to a delivery portion that may be separated or detached from the lead once it is positioned and/or anchored. For example, a delivery portion may include an elongate member having a control for controlling engagement of the anchors.

In operation, the lead may be positioned using bimanual manipulation (pulling from both the distal and proximal ends) to optimize the implantation/insertion position. The lead may be activated during the implantation procedure in order to determine which implantation locations work best Once an optimal position has been determined, the anchors securing the device in position (e.g., within the foramen, and/or to the pedicle(s) or lamina) may be engaged. The distal guidewire can be detached and removed. In some variations, the distal anchor may be activated (enraged) by detaching the guidewire/pullwire from the distal end of the lead.

Also described herein are exemplary methods of treating or preparing one or more joints. For example, the devices, systems and methods described herein may be used to resurface a joint, including resurfacing of cartilage and preparation for fusion of the joint. For example, a probe may be used to insert a guidewire between the sides or walls of a joint (e.g., a bone joint). As before, the wire may extend from a first (proximal) site through the body around and/or through the joint, and out of a second (distal) site of the body, allowing bimanual control. A tissue modification device that is configured to resurface the joint may then be coupled to the distal end of the guidewire/pullwire and pulled into position within the joint and used to resurface the joint.

For example, in one variation, the methods and systems described herein include facet joint fusion methods and systems. A facet joint may be fused by first accessing the joint, then preparing the joint and particularly the joint surface(s) (e.g., by roughening or abrading). The joint may then be fixed using a support (e.g., a cage, etc.) or a settable material (bone cement) or graft material. In some variations the fixation step (which may be optional) may include pulling an expandable or fillable material into position and expanding and/or filling it with material.

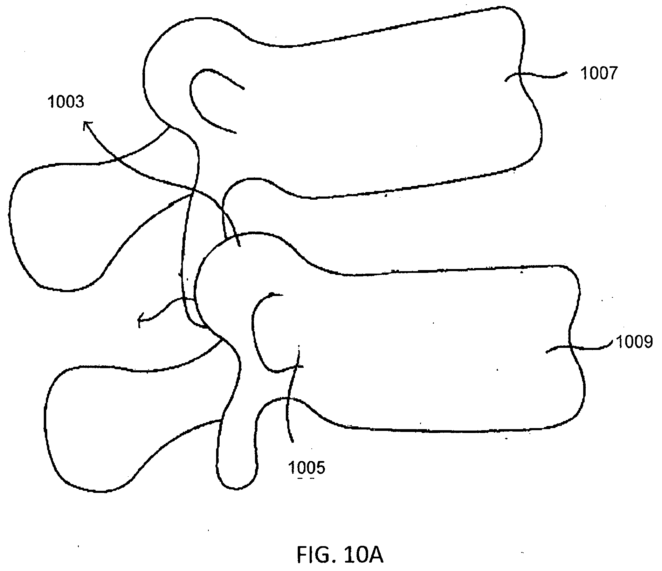

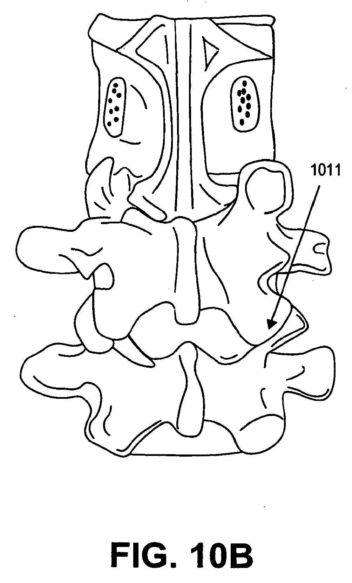

In one variation of a method for fusing a facet joint, a cannulated probe for guiding a guidewire/pullwire is first inserted in and/or around the joint. FIG. 10A illustrates a facet joint 1005 including the superior and inferior surfaces between the lower 1009 and upper 1007 vertebra. A guidewire/pullwire may be threaded through the facet joint as indicated by the line 1003. In some variations, the pathway through the facet joint passes over the top of the superior articulating process (SAP). In some variations, the pathway through the facet joint passes under the SAP giving access to the tip of the SAP. Placement of the guidewire around or through the facet joint may be aided by distraction of the spinous process, as described above. Thus, in some variations, the spinous process may be distracted before performing the procedure. FIG. 10B shows another portion of a spine including a facet joint 1011 that may be fused as described herein.

Once the probe has been used to position the guidewire, it may be removed. As illustrated above, the probe may include one or a plurality of (concentric) carmula including cannulas having different curvatures so that the guidewire may be directed around the joint and pointed toward the appropriate exit site. The guidewire or pullwire may then be pushed through the cannula and out of the patient. A distal handle may then be attached to the distal end of the guidewire to aid in manipulating the guidewire/pullwire from the distal end.

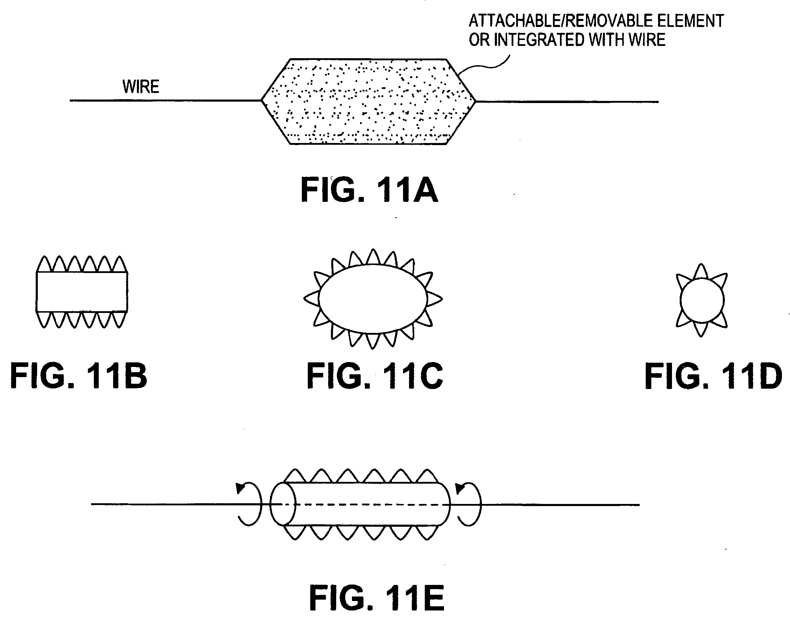

Next, a treatment device may be pulled into position in the joint by coupling the distal end (or end region) of the joint treatment device to the proximal end of the guidewire/pullwire. In some variations the treatment device includes one or more surfaces that are configured to abrade, scratch or otherwise prepare the surface for the fusion. For example, FIGS. 11A-11E illustrate variations of a treatment device. In FIG. 11A, the treatment device includes a front and a back articulating surface that can be drawn across the joint surfaces to roughen them. In this example, the distal end of the device includes an attachment/connector site for the guidewire. The proximal end also includes an elongate member and may have a proximal handle. In some variations the roughening surface is expandable, so that it may be pulled into the joint in a collapsed or condensed form (protecting non-target tissue), and once in the joint it can be expanded to the treatment form. For example, the device may be inflatable; inflation may expand the device so that the contact surface(s) can push against the joint surface(s). Once in position, the device can be moved bimanually within the joint to scrape or otherwise modify the joint surfaces, by pulling distally and proximally (e.g., back and forth).

FIGS. 11B-11E illustrate alternative cross-sections for the joint treatment devices described. For example, in FIG. 11B, the device is substantially flat, having an upper and lower surface. As mentioned, this device may be inflatable/expandable to increase (or decrease) the spacing between the upper and lower surfaces, or to "stiffen" the implant once it is expanded. FIG. 11C shows a device having an oval cross-section, and FIG. 11D shows a device having a round cross-section. In all of these variations the devices include 'teeth' or protrusions that are configured to roughen the joint surface, which may help with the fusion. In some variations the devices are configure to abrade, cut, and/or remove cartilage in the joint. In some variations the device is configured to abrade cartilage without substantially cutting or removing bone. In some variations the device surface is configured to abrade cut and/or remove bone from the joint.

The device may be actuated by moving it backwards and forwards (proximally and distally), by bimanual reciprocation. In some variations, such as that shown in FIG. 11E, for example, the device may also or alternatively be articulated by rotating it axially once it is in position in the joint.

In some variations the procedure for fusing the joint (e.g., facet joint) may include the use of more than one facet joint treatment devices. For example, treatment devices having different profiles (e.g., widths) may be used during the treatment. Alternatively, treatment may include selectively removing some of the bone or other tissue from the joint, which may be performed using the treatment device shown or using additional devices, including flexible bone biting devices such as the flexible ronguers described, for example in US 2007-0213733 A1, titled "Mechanical Tissue modification devices and methods" (filed 4/17/06). The same guidewire/pullwire may be used with multiple devices, as each device typically includes a distal coupler for securely coupling to the proximal end of the guidewire/pullwire, allowing it to be articulated within the joint.

Once the joint has been prepared using the device or devices, the device may be removed, and a support structure or material may be added to fuse the joint The guidewire/pullwire may remain in position, so that it can be used to pull in or apply the material. For example, in some variations the pullwire may be used to position a cage or other mechanical support within the joint The mechanical support may be coupled to the proximal end of the pullwire directly or indirectly (e.g., via an elongate carrier structure from which it can be released once it is positioned), and pulled into position. In some variations the pullwire may be used to pull a tube or other fluid material delivery device into position in the joint, to apply a filer material such as bone cement, bone graft material, etc. In some variations, the pullwire may be used to pull into position in the joint an expandable or fillable structure that will be implanted in the joint. For example, a mesh or porous "bag" structure may be pulled into position (and decoupled from the pullwire) and filled with appropriate fusing material (e.g., cement, etc.). In some variations a bag or balloon-like structure is pulled into position and filled.

As mentioned above, in any of the exemplary facet joint procedures described herein, all or a portion of the facet (e.g., the superior and/or inferior spinous processes) may be cut. For example, a procedure for fusing or preparing a facet joint may include a facetectomy, particularly for TLIF (Transforaminal Lumbar Interbody Fusion) procedures. The procedure may include a facet joint treatment device that is configured to saw through bone. For example, the device may include one or more cable-type saws including a distal end that is configured to couple to the pullwire as described above. As mentioned, a probe or probes may be used to place the pullwire under the facet joint A facet joint modifying device may then be pulled in under bimanual control. Pulling the facet joint modifying device dorsally (e.g., by distal/proximal reciprocation) would result in the removal of the entire facet joint. This method may be faster than current methods which involve slow biting with ronguer-type devices.

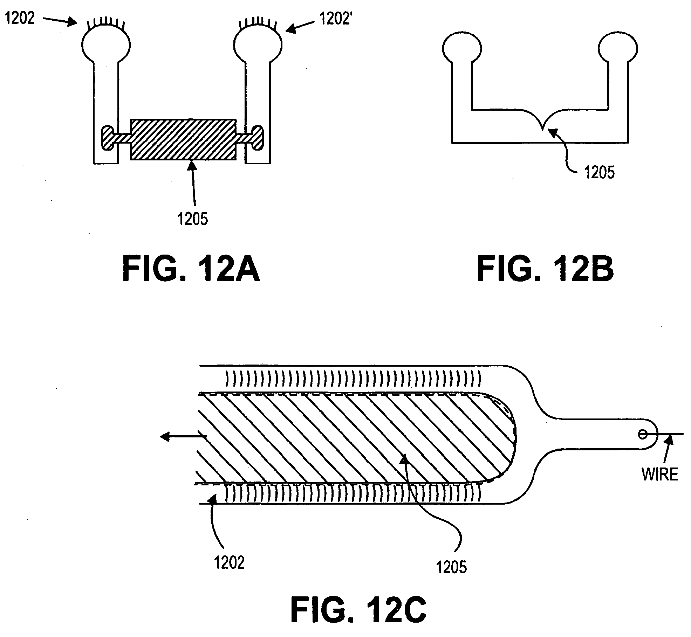

For example, FIG. 12A illustrates a cross-section through one variation of a facet-joint modifying device that includes two bone-sawing elements 1202, 1202'. The two saw elements (which may be cables or surfaces including blades) may be separated by a spacer 1205. FIG. 12C shows a top view of one variation of a facet-joint modifying device configured to perform a facetectomy. The distal end of the device is configured to couple with the pullwire, as described above. The tissue-contacting portion of the device may include two parallel cutting surfaces (which may be cables) 1202, 1202' that are separated from each other. These two separate cutting surfaces may allow two cuts to be made through the facet joint simultaneously, permitting removal of a portion of the facet joint. This version of the facet-joint modifying device may also include one or more spacers 1205. Spaces may prevent the cutting surfaces from spreading or contracting towards each other, particularly if the cutting surfaces are cables. In some variations these spacers may be removable or separating, so that as the facet joint modifying device cuts the facet joint, pressure applied as that device is reciprocated against the bone may cause separation, breaking, or removal of the spacer. FIG. 12B illustrates a cross-section through one portion of the device having a breakable (e.g., frangible) spacer 1205.

Other facet joint modifying devices (including those shown above in FIGS. 11A-11E) may include a single tissue-modifying surface, and thus does not need a spacer.

Also described herein are exemplary methods and systems for removing material from a body region, including removal of disc material. For example, the systems and devices describe herein may be used to perform discectomy and/or remove or repair of disc herniation.

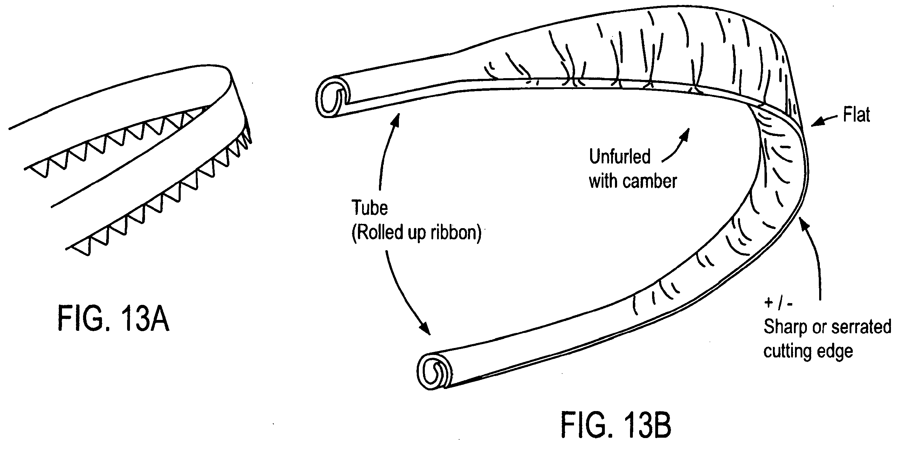

In disc treatments, a probe may be used to pass one or more guidewire/pullwires through the disc so that the guidewire/pullwire extends proximally from a proximal exit site around or through a portion of the disc, and out of the patient at a second, distal site. The guidewire is typically left in place while the probe may be removed. Once the guidewire/pullwire is in position, it may be used with one or more disc treatment devices. Examples of treatment devices are illustrated in FIGS. 13A-13B. these exemplary disc treatment devices are configured to be delivered relatively flexible into the disc region, but may be expanded or otherwise allowed to conform to a more rigid form once within the disc region. In FIG. 13A the curved device includes a serrated edge. The device may be a ribbon of material (including metal materials) that is stiffer when curved slightly than when allowed to lay flat. Another example of this variation is shown in FIG. 13B.

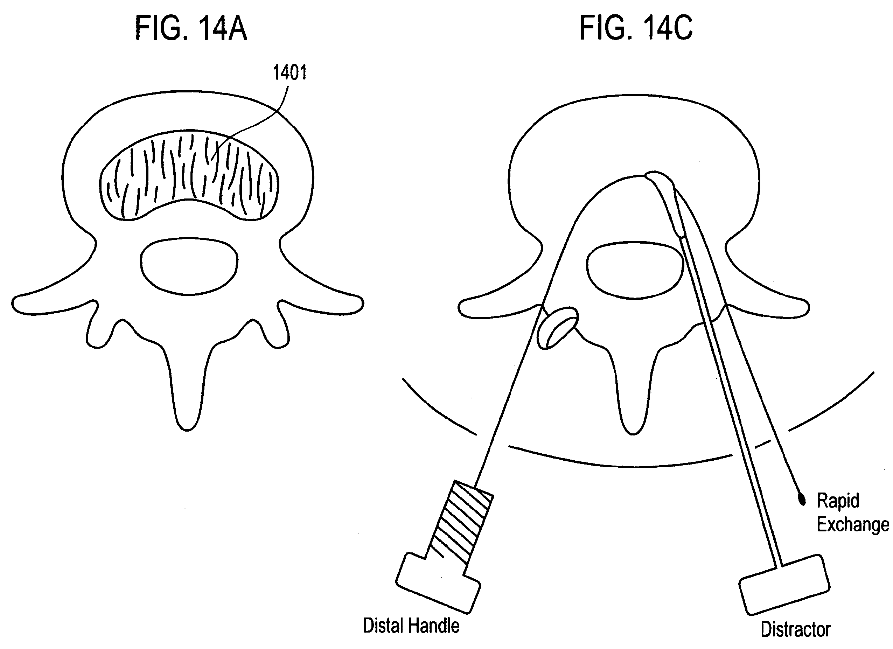

The systems and methods described herein may also be used as port of a Posterior Lumbar interbody Fusion (PLIF) procedure. Unilateral posterior or posteriorlateral approaches to access the disc space can be less invasive than bilateral approaches but instrument and implant positioning can be challenging. For example, it may be difficult to compete a discectomy contralaterally and position a single TLIF cage or posterior disc replacement across the appropriate disc space, as illustrated in FIG. 14A. The endplates are heterogeneous, and thus misplaced implants may not have the best contact with dense cortical bone 1401, placing them at greater risk for subsidence. To address this issue, a bimanually controlled pullwire system can be used to guide and pull instruments and implants into proper position in the disc space.

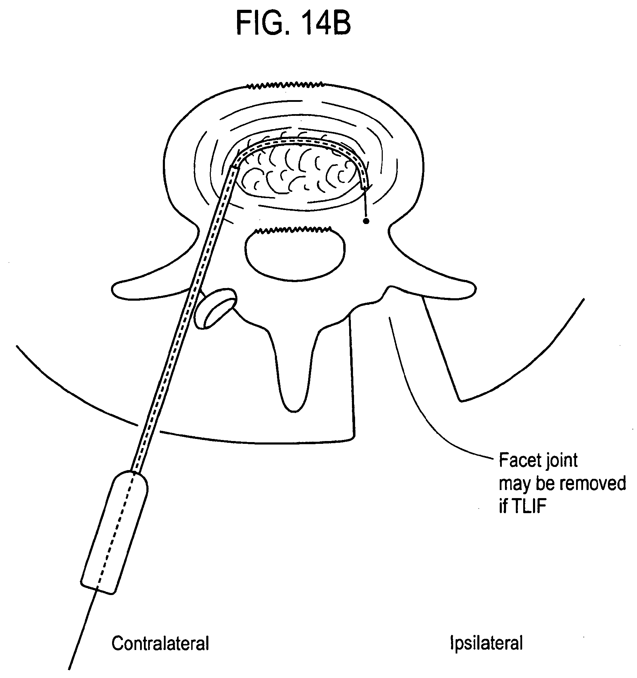

For example, FIGS. 14B and 14C illustrate one variation of a PLIF-type procedure that is made more effective using the pullwire techniques described herein. In FIG., 14B, for example, the guidewire/pullwire is first positioned in the disc space using a probe or probes, as described above. In one variation, a cannulated probe having a curved distal end is inserted contralaterally and percutaneously in to the disc space, toward an ispilateral direction. The pullwire may then be passed through the probe and out of the disc on the ipsilateral side. As illustrated in FIG., 14B, the procedure may be combined with a TLIF procedure in which part of the ipsilateral facet joint has been removed.

The pullwire may then be placed through the probe and extended distally out of the disc. In FIG. 14B, the pullwire extends distally from the ipsilateral incision (the excised region). Once the pullwire is in position, it may be used to pull one or more instruments or device (e.g., implants, cages, fillable/expandable structures, etc.) into place, as described above. In some variations a spacer or distractor may be used to open the disc space, as illustrated in FIG. 14C.

In any of the variations described herein, the exemplary method may also include the insertion of a pivot that may help guide the pullwire and/or devices pulled by the pullwire. For example, in FIG. 14C the distractor element may act as a pivot point to help control the ventral/dorsal location of the pullwire as it is manipulated. In this variation, the distractor is a pivot that is configured as a "rapid exchange" elongate element; the distal end of the pivot is configured to couple with the pullwire so that it can be pushed along the pullwire, yet still allow the pullwire to be pulled distally and proximally through or around the pivot In some variations the pivot includes a distal channel for the pullwire. As illustrated in FIG. 14C, the pivot may be inserted from either the proximal or distal end of the pullwire (typically after it has been initially positioned using the probe) and slide along the pullwire until it is positioned at the desired pivot point. Once in position, it may be held in place (e.g., anchored) from within the body or from outside of the body (e.g., by a clamp or other anchor), or simply held in place. In the variation shown in FIG. 14C, the pivot is also a distractor, and thus may be used to separate tissues (e.g., bone). In other variations the pivot is anchored or anchorable in the body, and provides a surface against which the pullwire may move without allowing substantial migration of the pullwire from the pathway through the body.

The examples and illustrations included herein show, by way of illustration and not of limitation, specific examples in which the subject matter may be practiced. Other examples may be utilized and derived there from, such that structural and logical substitutions and changes may be made without departing from the scope of the invention as defined in the appended claims.

## Claims

1. A system for inner spinous distraction, the system comprising:
an inner spinous distractor configured to be pulled into position through the inner spinous ligament (101) between two spinous processes (103, 103') and to distract the two spinous processes (103, 103');
a pullwire having a tissue-penetrating distal end and a coupler (507) at the proximal end, the coupler (507) configured to releasably couple to the inner spinous distractor so that the pullwire may be used to pull the inner spinous distractor into position; and
a cannulated probe configured to position the pull wire between two spinous processes.

2. The system of claim 1, further comprising a sizer (509) configured to couple to the proximal end of the pullwire so that it can be pulled between two spinous processes.

3. The system of claim 1 or 2, further comprising a distal handle (505) configured to attach to the distal end of the pullwire and to secure the tissue-penetrating distal end of the pullwire.

4. The system of any preceding claim, further comprising an inner spinous distractor delivery tool (510) configured to hold the inner spinous distractor for delivery between two spinous processes (103, 103'), wherein the distal end of the delivery tool (510) comprises a coupler for coupling to the proximal end of the pullwire and the proximal end of the inner spinous distractor delivery tool (510) comprises a proximal handle.

5. The system of any preceding claim, further comprising a lock for securing the inner spinous distractor in position between two spinous processes.

## Patentansprüche

1. System für interne Dornfortsatzdistraktion, wobei das System Folgendes umfasst:
einen internen Dornfortsatzdistraktor, konfiguriert, um durch das Ligamentum interspinale (101) zwischen zwei Dornfortsätzen (103, 103') in Position gezogen zu werden und die zwei Dornfortsätze (103, 103') auseinanderzubewegen;
ein Zugseil mit einem gewebedurchdringenden distalen Ende und einer Kupplung (507) am proximalen Ende, wobei die Kupplung (507) konfiguriert ist, um lösbar derart an den internen Dornfortsatzdistraktor zu koppeln, dass das Zugseil verwendet werden kann, um den internen Dornfortsatzdistraktor in Position zu ziehen; und
eine durchbohrte Sonde, konfiguriert, um das Zugseil zwischen zwei Dornfortsätzen zu positionieren.

2. System nach Anspruch 1, ferner umfassend eine Größenmessvorrichtung (509), konfiguriert, um an das proximale Ende des Zugseils zu koppeln, sodass sie zwischen zwei Dornfortsätze gezogen werden kann.

3. System nach Anspruch 1 oder 2, ferner umfassend einen distalen Griff (505), konfiguriert, um an dem distalen Ende des Zugseils angebracht zu werden und das gewebedurchdringende distale Ende des Zugseils zu sichern.

4. System nach einem der vorhergehenden Ansprüche, ferner umfassend ein Einsatzwerkzeug (510) für einen internen Dornfortsatzdistraktor, konfiguriert, um den internen Dornfortsatzdistraktor für das Einsetzen zwischen zwei Dornfortsätzen (103, 103') zu halten, wobei das distale Ende des Einsatzwerkzeugs (510) eine Kupplung zum Koppeln an das proximale Ende des Zugseils umfasst und das proximale Ende des Einsatzwerkzeugs (510) für einen internen Dornfortsatzdistraktor einen proximalen Griff umfasst.

5. System nach einem der vorhergehenden Ansprüche, ferner umfassend eine Verriegelungsvorrichtung zum Sichern des internen Dornfortsatzdistraktors zwischen zwei Dornfortsätzen.

## Revendications

1. Système destiné à réaliser une distraction épineuse interne, le système comportant :
un distracteur épineux interne configuré pour être tiré en position à travers le ligament (101) épineux interne entre deux apophyses (103, 103') épineuses et pour réaliser la distraction des deux apophyses (103, 103') épineuses ;
un fil métallique de traction présentant une extrémité distale pénétrant dans les tissus et un coupleur (507) au niveau de l'extrémité proximale, le coupleur (507) étant configuré pour s'accoupler de manière libérable au distracteur épineux interne de façon à ce que le fil métallique de traction puisse être utilisé pour tirer le distracteur épineux interne en position ; et
une sonde à canule configurée pour positionner le fil métallique de traction entre deux apophyses épineuses.

2. Système selon la revendication 1, comportant en outre un dispositif de dimensionnement (509) configuré pour s'accoupler à l'extrémité proximale du fil métallique de traction de façon à pouvoir être tiré entre deux apophyses épineuses.

3. Système selon la revendication 1 ou 2, comportant en outre une poignée (505) distale configurée pour se fixer à l'extrémité distale du fil métallique de traction et pour immobiliser l'extrémité distale pénétrant dans les tissus du fil métallique de traction.

4. Système selon l'une quelconque des revendications précédentes, comportant en outre un outil (510) de mise en place de distracteur épineux interne configuré pour retenir le distracteur épineux interne afin de le mettre en place entre deux apophyses (103, 103') épineuses, l'extrémité distale de l'outil (510) de mise en place comportant un coupleur destiné à s'accoupler à l'extrémité proximale du fil métallique de traction et l'extrémité proximale de l'outil (510) de mise en place de distracteur épineux interne comporte une poignée proximale.

5. Système selon l'une quelconque des revendications précédentes, comportant en outre un verrou destiné à immobiliser le distracteur épineux interne en position entre deux apophyses épineuses.
